# EUROPEAN PATENT APPLICATION

(11) **EP 3 211 862 A1**
(43) Date of publication of application: **30.08.2017**
(21) Application number: 17157083.1
(22) Date of filing: 21.02.2017
(51) Int. Cl.: H04L 29/08, H04L 29/06, G06F 3/0488

(54) **INTEGRATION PLATFORM OF INTERNET OF THINGS**

(30) Priority: 25.02.2016 TW 016202634 U
(71) Applicant: Hsiao, Jue-Hsuan, New Taipei City 220 (TW)
(72) Inventor: Hsiao, Jue-Hsuan, New Taipei City 220 (TW)
(74) Representative: Becker Kurig Straus

(57) **Abstract**

An integration platform of Internet of Things is provided. An integration platform of Internet of Things comprises a master apparatus, at least one first IOT object and a vendor-side server. The first IOT object comprises a first transmitting and receiving unit and a first device body. The first transmitting and receiving unit is connected to the master apparatus. The first device body is connected to the first transmitting and receiving unit via a way of a wireless communication. The vendor-side server is connected to the master apparatus via a way of a telecommunication. The first cloud simulation object is stored in the vendor-side server. The appearance of the first cloud simulation object is corresponding to an appearance of the first device body. When the first transmitting and receiving unit is connected to the master apparatus for the first time, a first cloud simulation object is transmitted into the controlling map by the vendor-side server. Wherein controlling the first cloud simulation object of the controlling map would operate the first device body corresponding to the first cloud simulation object.

## Description

### FIELD OF THE INVENTION

The present invention relates to an integration platform of Internet of Things, more particularly to an integration platform of Internet of Things having a controlling map and a measurement list.

### BACKGROUND OF THE INVENTION

With the evolution of the times, mobile communications and Internet are becoming more and more popular. Recently a new concept is presented known as Internet of Things (IOT). Machinery, equipment, personnel could be centrally managed and controlled via the Internet of Things. For example, home electronics(IOT objects), vehicles and other means of transport, physiological signal monitoring equipment for medical, a search for a location, preventing items from being stolen and other items.

However, on the uses of the Internet of Things of today, aside from the kind of the IOT object being few, the IOT object of various vendors does not have a consistent control mode. Therefore, it is not convenient to operate the IOT object, and the user does not have many choices. Specifically, because a control method of each IOT object are different, a user must adapt a user interface of each IOT object (Usually each IOT object include a dedicated user interface or mobile application). As a result, the user would not want to buy IOT object (Different user interfaces would cause large trouble with the user.), and the integration and development of Internet of Things at home environment or in the working environment could not be improved.

Therefore, how to integrate the IOT objects of various vendors and provide a safe, convenient, and friendly user interface is worth considering to a person having ordinary skills in the art.

### SUMMARY OF THE INVENTION

An integration platform of Internet of Things is provided. The integration platform of Internet of Things is configured to integrate the IOT objects of various vendors, and the integration platform of Internet of Things includes user-friendly control in order to create the user's a smart home environment or a smart working environment.

An integration platform of Internet of Things is provided. An integration platform of Internet of Things comprises a master apparatus, at least one first IOT object and a vendor-side server. The first IOT object comprises a first transmitting and receiving unit and a first device body. The first transmitting and receiving unit is connected to the master apparatus. The first device body is connected to the first transmitting and receiving unit via a way of a wireless communication. The vendor-side server is connected to the master apparatus via a way of a telecommunication (e.g., mobile, mobile communications, wired or wireless network). The first cloud simulation object is stored in the vendor-side server. The appearance of the first cloud simulation object is corresponding to an appearance of the first device body. When the first transmitting and receiving unit is connected to the master apparatus for the first time, a first cloud simulation object is transmitted into the controlling map by the vendor-side server. Wherein controlling the first cloud simulation object of the controlling map would operate the first device body corresponding to the first cloud simulation object.

In the integration platform of Internet of Things, an operating status of the first cloud simulation object of the controlling map is the same as an operating status of the first device body corresponding to the first cloud simulation object.

In the integration platform of Internet of Things, the integration platform further comprises a client server. The controlling map is transmitted into the client server via the way of the telecommunication by the master apparatus. The controlling map wirelessly is transmitted into at least one portable electronic device by the client server.

In the integration platform of Internet of Things, controlling the first cloud simulation object of the controlling map of the portable electronic device would operate the first device body corresponding to the first cloud simulation object.

In the integration platform of Internet of Things, the appearance of the first cloud simulation object is three-dimensional graphics or plane graphics.

In the integration platform of Internet of Things, the controlling map is a home environment simulating diagram or a working environment simulating diagram.

In the integration platform of Internet of Things, the integration platform further comprises at least one second IOT object. The second IOT object comprises a second transmitting and receiving unit connected and a physiological measurement body. The second transmitting and receiving unit is connected to the master apparatus. The physiological measurement body is connected to the second transmitting and receiving unit via a way of a wireless communication. A blood pressure, an ECG, a heart rate value, and a blood oxygen saturation value of a user are measured by the physiological measurement body.

In the integration platform of Internet of Things, the master apparatus further comprises a measurement list, and the blood pressure, the ECG, the heart rate value and the blood oxygen saturation value is transmitted into the measurement list by the second IOT object.

In the integration platform of Internet of Things, the measurement list is transmitted into the client server via the way of the telecommunication by the master apparatus. The measurement list is wirelessly transmitted into the portable electronic device by the client server.

In the integration platform of Internet of Things, a second cloud simulation object is stored in the vendor-side server. An appearance of the second cloud simulation object is corresponding to an appearance of the physiological measurement body.

In the integration platform of Internet of Things, when the second transmitting and receiving unit is connected to the master apparatus for the first time, a second cloud simulation object is transmitted into the controlling map by the vendor-side server.

In the integration platform of Internet of Things, when the second cloud simulation object of the controlling map is touched, the physiological measurement body corresponding to the second cloud simulation object would start working.

In the integration platform of Internet of Things, the integration platform further comprises at least one vendor apparatus. The vendor apparatus is connected to the vendor-side server via the way of the telecommunication. The vendor apparatus includes an object design module, the first cloud simulation object and the second cloud simulation object is created at the object design module. The first cloud simulation object and the second cloud simulation object is transmitted into the vendor-side server by the vendor apparatus.

In the integration platform of Internet of Things, the portable electronic device is a smart phone, a tablet, a laptop, or a VR glasses.

In the integration platform of Internet of Things, the first device body is a fan, an air conditioning, a heating, a TV, a fridge, a game console, a washing machine, an electric water boiler, a gas stove, a range hood or a water heater.

The foregoing, as well as additional objects, features and advantages of the invention will be more readily apparent from the following detailed description, which proceeds with reference to the accompanying drawings.

### BRIEF DESCRIPTION OF THE DRAWINGS

Fig.1A illustrates an integration platform 10 of Internet of Things with a first embodiment of the present invention;
Fig.1B illustrates a block diagram of the integration platform 10 of Internet of Things;
Fig.2 illustrates a schematic diagram of a controlling map 110;
Fig.3 illustrates a schematic diagram of a first cloud simulation object 122C being in the controlling map 110;
Fig.4 illustrates a schematic diagram of the first cloud simulation object 122C operated in the controlling map 110;
Fig.5A illustrates an integration platform 20 of Internet of Things with a second embodiment of the present invention;
Fig.5B illustrates a block diagram of the integration platform 20 of Internet of Things;
Fig.6 illustrates a schematic diagram of a user measured by a physiological measurement body 232;
Fig.7 illustrates a schematic diagram of a measurement list 210 displayed on a portable electronic device 16;
Fig.8 illustrates a schematic diagram of a second cloud simulation object 222C being in the controlling map 110.

### DETAILED DESCRIPTION OF PREFERRED EMBODIMENTS

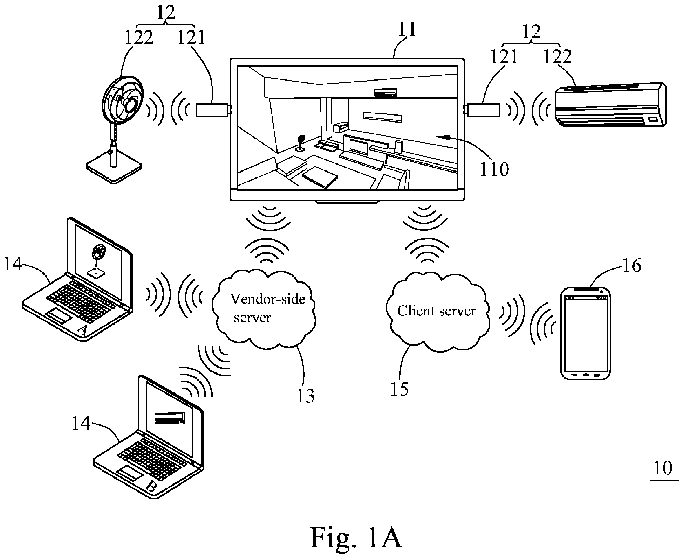

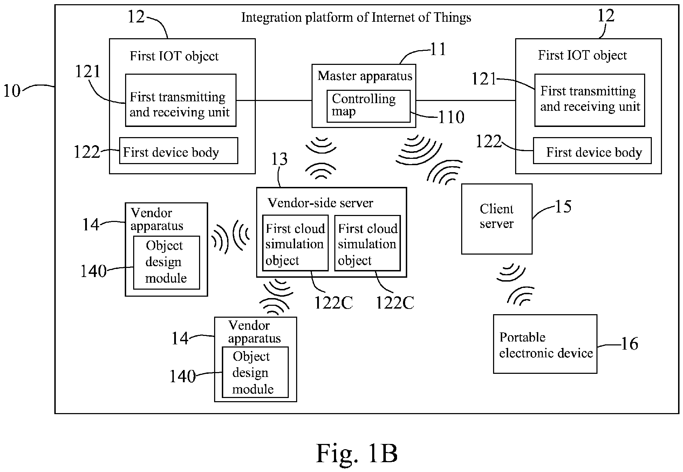

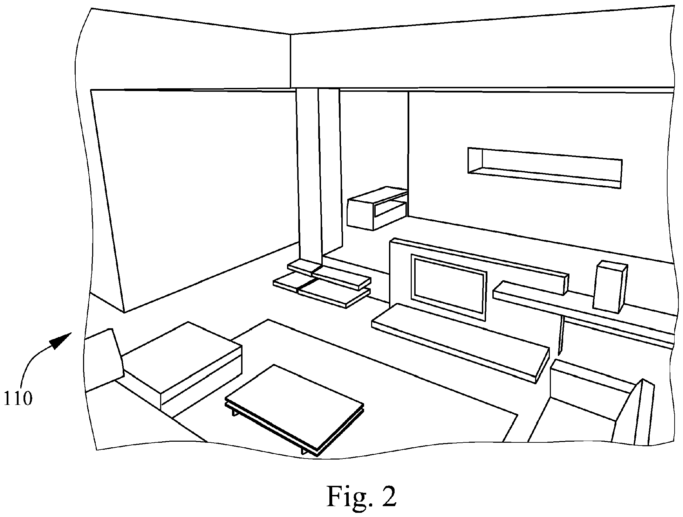

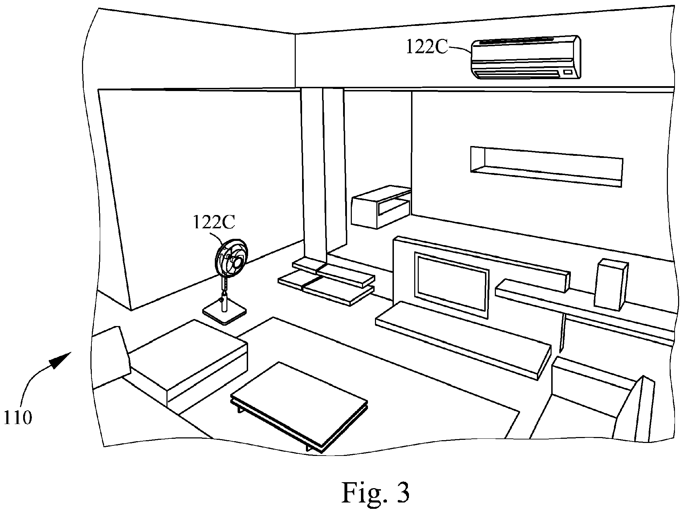

Please refer to Fig.1A and Fig.1B. Fig.1A illustrates an integration platform 10 of Internet of Things with a first embodiment of the present invention. Fig.1B illustrates a block diagram of the integration platform 10 of Internet of Things. The integration platform 10 of Internet of Things includes a master apparatus 11, at least one first IOT object 12, a vendor-side server 13, at least one vendor apparatus 14, a client server 15 and at least one portable electronic device 16. The master apparatus 11 includes at least one controlling map 110. The controlling map 110 is a home environment simulating diagram or a working environment simulating diagram (Please refer to Fig. 2. The home environment simulating diagram is as a sample for the controlling map 110 of the first embodiment.). The home environment simulating diagram is created according to imitating a home environment of a user of the master apparatus 11, and the working environment simulating diagram is created according to imitating a working environment of a user of the master apparatus 11. Furthermore, the first IOT object 12 includes a first transmitting and receiving unit 121 and a first device body 122. The first transmitting and receiving unit 121 is connected to the master apparatus 11. For example, the first device body 122 is a fan, an air conditioning, a heating, a TV, a fridge, a game console, a washing machine, an electric water boiler, a gas stove, a range hood, a water heater or other home appliances. The first device body 122 includes a wireless communications chip, so the first device body 122 could be connected to the first transmitting and receiving unit 121 via a way of a wireless communication. In other words, the first transmitting and receiving unit 121 is a bridge between the master apparatus 11 and the first device body 122. For example, the way of wireless communication is a Wi-Fi transmission or a Bluetooth transmission. In the first embodiment of the present invention, two first IOT objects 12 are used. One is produced by a manufacturer A (the manufacturer A is a manufacturer of fans.), and the other is produced by a manufacturer B (the manufacturer B is a manufacturer of air conditionings.). The first device body 122 of the manufacturer A is a fan, and the first device body 122 of the manufacturer B is an air conditioning. In addition, the vendor-side server 13 is connected to the master apparatus 11 and the vendor apparatus 14 via the way of the wireless communication. Moreover, in the first embodiment of the present invention, two vendor apparatuses 14 are used. One is computer equipment used by the manufacturer A, and the other is computer equipment used by the manufacturer B. Furthermore, each vendor apparatuses 14 further includes an object design module 140. A first cloud simulation object 122C is created at the object design module 140. It is worth noting that an appearance of the first cloud simulation object 122C of each vendor apparatuses 14 is corresponding to an appearance of the first device body 122 of each vendor apparatuses 14. For example, if the first device body 122 produced by the manufacturer A is a fan, the appearance of the first cloud simulation object 122C of the manufacturer A should be three-dimensional graphics or plane graphics of the fan. Similarly, if the first device body 122 produced by the manufacturer B is an air conditioning, the appearance of the first cloud simulation object 122C of the manufacturer B should be three-dimensional graphics or plane graphics of the air conditioning. Then, the first cloud simulation object 122C of the manufacturer A is transmitted into the vendor-side server 13 by the vendor apparatuses 14 of the manufacturer A, and the first cloud simulation object 122C of the manufacturer B is also transmitted into the vendor-side server 13 by the vendor apparatuses 14 of the manufacturer B. When the first transmitting and receiving unit 121 of the manufacturer A is connected to the master apparatus 11 for the first time, a first link signal is sent to the vendor-side server 13 by the master apparatus 11. Then, a first cloud simulation object 122C (a three-dimensional graphic of the fan) is transmitted into the controlling map 110 of the master apparatus 11 by the vendor-side server 13 (Please refer to Fig.3. Fig.3 illustrates a schematic diagram of the first cloud simulation object 122C being in the controlling map 110.). Similarly, when the first transmitting and receiving unit 121 of the manufacturer B is connected to the master apparatus 11 for the first time, a first cloud simulation object 122C (a three-dimensional graphic of the air conditioning) is also transmitted into the controlling map 110 of master apparatus 11 by the vendor-side server 13.

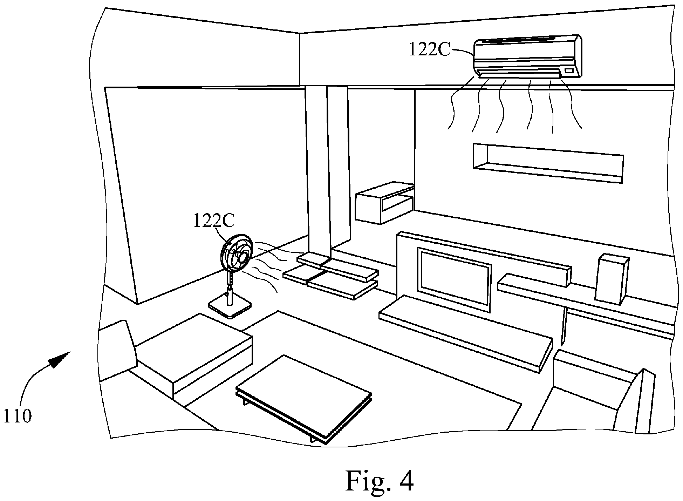

Afterward, when the user of the master apparatus 11 controls the first cloud simulation object of the controlling map, the user would indirectly operate the first device body 122 corresponding to the first cloud simulation object 122C. As a result, an operating status of the first cloud simulation object 122C of the controlling map 110 is the same as an operating status of the first device body 122 corresponding to the first cloud simulation object 122C. In more detail, when the user touches the first cloud simulation object 122C of the controlling map 110 (e.g., touching the three-dimensional graphic of the fan), the first cloud simulation object 122C would begin the transition from a stationary state to an operational state. In other word, the three-dimensional graphic of the fan would show flow of wind (Please refer to Fig.4. Fig.4 illustrates a schematic diagram of the first cloud simulation object 122C operated in the controlling map 110.). At the same time, the first device body 122 would also begin the transition from a stationary state to an operational state. Specifically, the real fan has been turned on. Similarly, please refer to Fig.4 again. When the user touches another first cloud simulation object 122C of the controlling map 110 (e.g., touching the three-dimensional graphic of the air conditioning), the real air conditioning has been turned on. As a result, a user interface of the first IOT object 12 of various vendors (the manufacturer A and the manufacturer B) is integrated in the controlling map 110 of the master apparatus 11 (The first cloud simulation object 122C is configured to control the first device body 122.). Therefore, when the user of the master apparatus 11 uses the IOT object of various vendors, the IOT object of various vendors has a consistent control mode. As a result, it is very convenient to use, simple and user-friendly operation. In the above, the user of the master apparatus 11 could modify detail status of the first cloud simulation object 122C via the controlling map 110 in order that the first cloud simulation object 122C is consistent with the real environment. For example, the detail status is a location or a proportion of the first cloud simulation object 122C.

Please refer to Fig.1 again. The client server 15 is connected to the master apparatus 11 and the portable electronic device 16 via the way of the telecommunication. For example, the portable electronic device is a smart phone, a tablet, a laptop, or a VR glasses (The smart phone is as a sample for the portable electronic device 16 in the first embodiment of the present invention.). In addition, the controlling map 110 of the first cloud simulation object 122C is transmitted into the client server 15 by the master apparatus 11. Then, the controlling map 110 is wirelessly transmitted into the portable electronic device 16 by the client server 15. As a result, even though a holder of the portable electronic device 16 is in the distance, the holder could get a state (on or off) of the first IOT object 12 via the controlling map 110 of the portable electronic device 16. Thus the holder effectively gets the actual situation at home. In the above, the controlling map 110 is only transmitted into one portable electronic device 16. However, the controlling map 110 could be transmitted into a plurality of portable electronic device 16 in order that the holder of more portable electronic devices 16 gets the situation at home. Furthermore, aside from getting the situation at home, the holder of the portable electronic devices 16 could also control the first cloud simulation object 122C of the portable electronic devices 16 in order to operate the first device body 122.

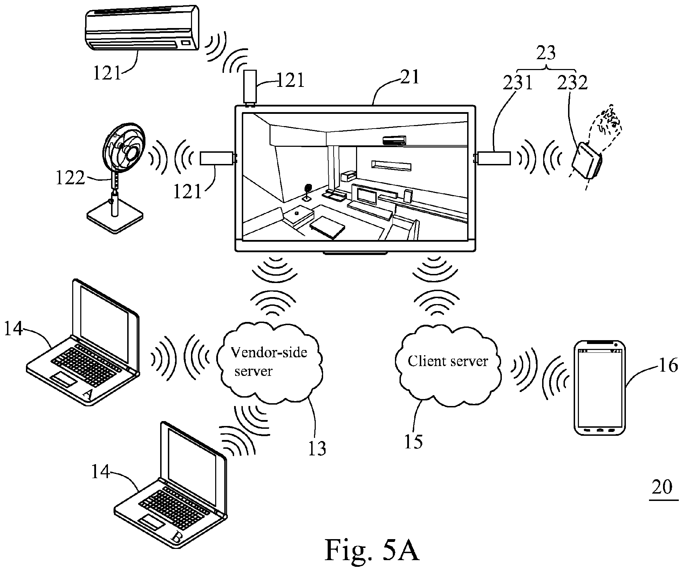

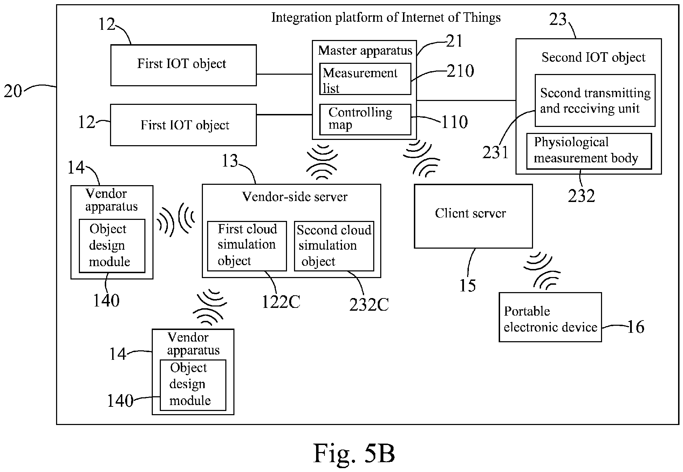

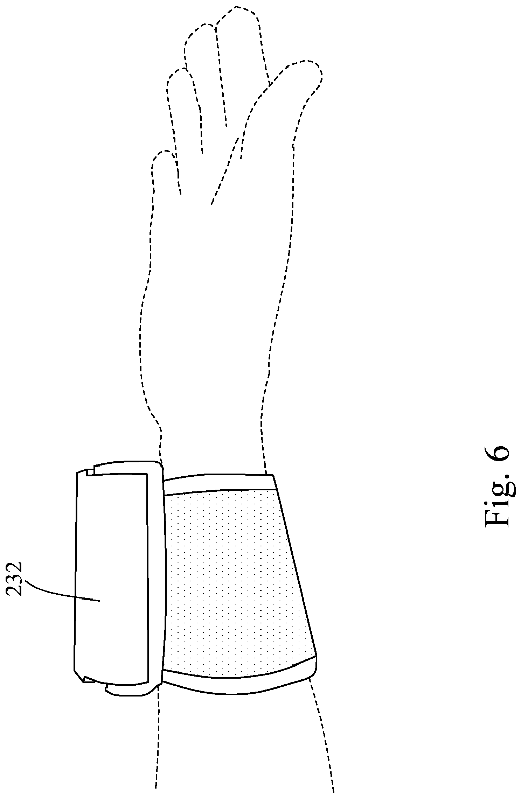

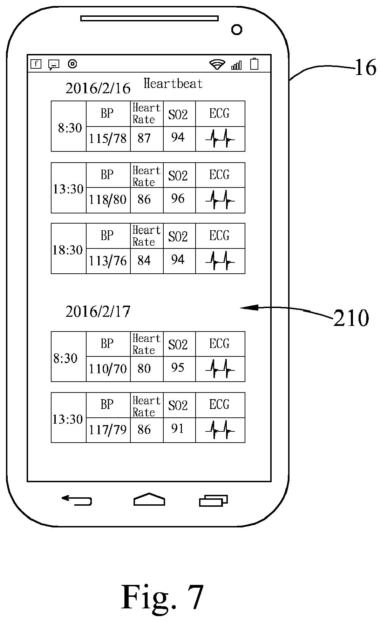

Please refer to Fig.5A and Fig.5B. Fig.5A illustrates an integration platform 20 of Internet of Things with a second embodiment of the present invention. Fig.5B illustrates a block diagram of the integration platform 20 of Internet of Things. The integration platform 20 is derived from the integration platform 10. The integration platform 20 further includes a second IOT object 23. The second IOT object 23 includes a second transmitting and receiving unit 231 and a physiological measurement body 232. The second transmitting and receiving unit 231 is connected to the master apparatus 21. The physiological measurement body 232 is connected to the second transmitting and receiving unit 231 via the way of the wireless communication. A blood pressure, an ECG, a heart rate value, and a blood oxygen saturation value of a user are measured by the physiological measurement body 232 (Please refer to Fig.6. Fig.6 illustrates a schematic diagram of a user measured by a physiological measurement body 232.). Moreover, the master apparatus further includes a measurement list 210. The blood pressure, the ECG, the heart rate value and the blood oxygen saturation value is transmitted into the measurement list 210 by the second IOT object 23. Then, the measurement list 210 is transmitted into the client server 15 via the way of the telecommunication by the master apparatus 21. Afterward, the measurement list 210 is wirelessly transmitted into the portable electronic device 16 by the client server 15. Therefore, both the user of the master apparatus 21 and the holder of more portable electronic devices 16 could get a variety of physiological values of the user of the physiological measurement body 232 via the measurement list 210. As a result, it is in favor of grasping health status of the user of the physiological measurement body 232. (Please refer to Fig.7. Fig.7 illustrates a schematic diagram of a measurement list 210 displayed on portable electronic device 16.)

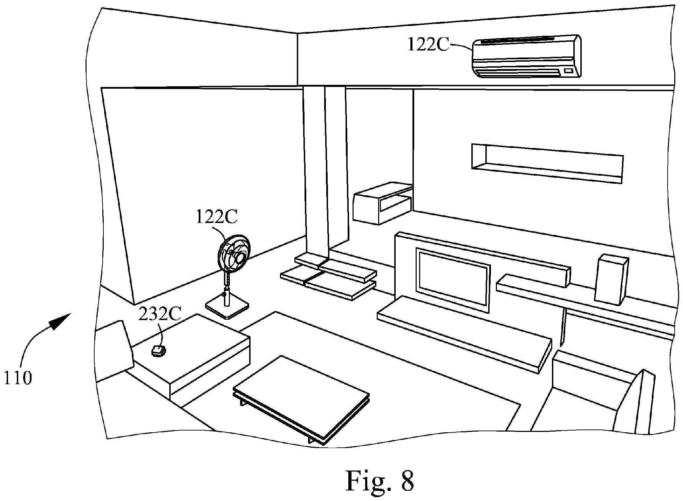

Furthermore, please refer to Fig.5B again. A second cloud simulation object 232C is stored in the vendor-side server 13. The second cloud simulation object 232C is created at the object design module 140. It is worth noting that an appearance of the second cloud simulation object 232C is corresponding to an appearance of the physiological measurement body 232. For example, if the physiological measurement body 232 produced by the manufacturer A is an instrument for measuring physiological values, the appearance of the second cloud simulation object 222C of the manufacturer A should be three-dimensional graphics or plane graphics of the instrument. When the second transmitting and receiving unit 31 is connected to the master apparatus 21 for the first time, a second link signal is sent to the vendor-side server 13 by the master apparatus 21. Then, a second cloud simulation object 232C (a three-dimensional graphic of the instrument) is transmitted into the controlling map 110 of the master apparatus 21 by the vendor-side server 13 (Please refer to Fig.8. Fig.8 illustrates a schematic diagram of the second cloud simulation object 122C being in the controlling map 110.). Then, when the user of the master apparatus 21 touches the second cloud simulation object 232C of the controlling map 110, the physiological measurement body 232 corresponding to the second cloud simulation object 232C would begin working. Specifically, the physiological measurement body 232 is configured to measure a variety of physiological values (e.g., the blood pressure, the heart rate value and the blood oxygen saturation value) of the user. As a result, the consistency and convenience of operating the IOT object for medical are ensured.

In summary, the integration platform 10 of Internet of Things or the integration platform 20 of Internet of Things could effectively integrate the IOT objects (e.g., the IOT object for medical, home environment and working environment) of various vendors and provide a consistent, safe, convenient user interface having plug and play function. In addition, from manufacturers' point of view, using the integration platform 10, 20 (open system) could help each manufacturer to develop more types of networking products. As a result, the user has many choices about the networking products, and the development of Internet of Things is more vigorous.

Although the description above contains many specifics, these are merely provided to illustrate the invention and should not be construed as limitations of the invention's scope. Thus it will be apparent to those skilled in the art that various modifications and variations can be made in the system and processes of the present invention without departing from the spirit or scope of the invention.

## Claims

1. An integration platform of Internet of Things comprising:
a master apparatus including a controlling map;
at least one first IOT object, the IOT object comprising:
a first transmitting and receiving unit connected to the master apparatus;
a first device body connected to the first transmitting and receiving unit via a way of a wireless communication;
a vendor-side server connected to the master apparatus via a way of a telecommunication, a first cloud simulation object stored in the vendor-side server, and an appearance of the first cloud simulation object is corresponding to an appearance of the first device body;
Wherein when the first transmitting and receiving unit is connected to the master apparatus for the first time, a first cloud simulation object is transmitted into the controlling map by the vendor-side server; wherein controlling the first cloud simulation object of the controlling map would operate the first device body corresponding to the first cloud simulation object.

2. The integration platform of Internet of Things of claim 1, wherein an operating status of the first cloud simulation object of the controlling map is the same as an operating status of the first device body corresponding to the first cloud simulation object.

3. The integration platform of Internet of Things of claim 2 further comprising a client server, the controlling map transmitted into the client server via the way of the telecommunication by the master apparatus, the controlling map wirelessly transmitted into at least one portable electronic device by the client server.

4. The integration platform of Internet of Things of claim 3, wherein controlling the first cloud simulation object of the controlling map of the portable electronic device would operate the first device body corresponding to the first cloud simulation object.

5. The integration platform of Internet of Things of claim 1, wherein the appearance of the first cloud simulation object is three-dimensional graphics or plane graphics.

6. The integration platform of Internet of Things of claim 1, wherein the controlling map is a home environment simulating diagram or a working environment simulating diagram.

7. The integration platform of Internet of Things of claim 3 further comprising at least one second IOT object, the second IOT object comprising:
a second transmitting and receiving unit connected to the master apparatus;
a physiological measurement body connected to the second transmitting and receiving unit via a way of a wireless communication;
wherein a blood pressure, an ECG, a heart rate value, and a blood oxygen saturation value of a user are measured by the physiological measurement body.

8. The integration platform of Internet of Things of claim 7, wherein the master apparatus further comprises a measurement list, and the blood pressure, the ECG, the heart rate value and the blood oxygen saturation value is transmitted into the measurement list by the second IOT object.

9. The integration platform of Internet of Things of claim 8, wherein the measurement list is transmitted into the client server via the way of the telecommunication by the master apparatus, and the measurement list is wirelessly transmitted into the portable electronic device by the client server.

10. The integration platform of Internet of Things of claim 9, wherein a second cloud simulation object is stored in the vendor-side server, and an appearance of the second cloud simulation object is corresponding to an appearance of the physiological measurement body.

11. The integration platform of Internet of Things of claim 10, wherein when the second transmitting and receiving unit is connected to the master apparatus for the first time, a second cloud simulation object is transmitted into the controlling map by the vendor-side server.

12. The integration platform of Internet of Things of claim 11, wherein when the second cloud simulation object of the controlling map is touched, the physiological measurement body corresponding to the second cloud simulation object would start working.

13. The integration platform of Internet of Things of claim 11 further comprising at least one vendor apparatus, and the vendor apparatus is connected to the vendor-side server via the way of the telecommunication, and the vendor apparatus includes an object design module, and the first cloud simulation object and the second cloud simulation object are created at the object design module, and the first cloud simulation object and the second cloud simulation object are transmitted into the vendor-side server by the vendor apparatus.

14. The integration platform of Internet of Things of claim 3, wherein the portable electronic device is a smart phone, a tablet, a laptop, or a VR glasses.

15. The integration platform of Internet of Things of claim 3, wherein the first device body is a fan, an air conditioning, a heating, a TV, a fridge, a game console, a washing machine, an electric water boiler, a gas stove, a range hood or a water heater.
